(19) **Europäisches Patentamt**
**European Patent Office**
**Office européen des brevets**

(11) **EP 4 098 291 B1**

(12) **EUROPEAN PATENT SPECIFICATION**

(45) Date of publication and mention
of the grant of the patent:
**21.01.2026 Bulletin 2026/04**

(21) Application number: **21176784.3**

(22) Date of filing: **31.05.2021**

(51) International Patent Classification (IPC):
**A61M 1/16** *(2006.01)*      **A61M 1/36** *(2006.01)*

(52) Cooperative Patent Classification (CPC):
**A61M 1/1603;** A61M 1/1607; A61M 1/1609;
A61M 1/1611; A61M 1/3612; A61M 2205/3306;
A61M 2205/331; A61M 2205/3331;
A61M 2205/3334; A61M 2205/3379; A61M 2230/04;
A61M 2230/20; A61M 2230/65

(54) **AN APPARATUS FOR EXTRACORPOREAL BLOOD TREATMENT**

VORRICHTUNG ZUR EXTRAKORPORALEN BLUTBEHANDLUNG

APPAREIL DE TRAITEMENT EXTRACORPOREL DU SANG

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**07.12.2022 Bulletin 2022/49**

(73) Proprietor: **Gambro Lundia AB
226 43 Lund (SE)**

(72) Inventors:
• **ROVATTI, Paolo
41034 Finale Emilia (Modena) (IT)**

• **SURACE, Alessandro
41012 Carpi (Modena) (IT)**

(74) Representative: **PGA S.p.A., Milano, Succursale
di Lugano
Via Castagnola, 21c
6900 Lugano (CH)**

(56) References cited:
**WO-A1-2012/172398**

Remarks:
The file contains technical information submitted
after the application was filed and not included in this
specification

Processed by Luminess, 75001 PARIS (FR)

**Description**

The present invention relates to an apparatus for extracorporeal blood treatment.

An apparatus for extracorporeal blood treatment comprises at least one treatment unit (for example a dialyser or a filter or ultrafilter or a plasma filter or a filtering unit of another type) having a semipermeable membrane which separates the treatment unit into two chambers. An extracorporeal blood circuit enables circulation of blood removed from a patient internally of the first chamber. At the same time and typically in a counter-current direction with respect to the blood, a treatment fluid is made to circulate through an appropriate circuit in the second chamber of the treatment unit. This type of apparatus for blood treatment, known as dialysis apparatus, may be used for removal of excess solutes and fluids from the blood of patients suffering from kidney failure. A particular type of apparatus for blood treatment, known as hemofiltration or hemodiafiltration apparatus, further comprises one or more infusion lines predisposed to send a replacement fluid into the extracorporeal blood circuit. The infusion line or lines are connected upstream and/or downstream with respect to the treatment unit. The above-described blood treatment apparatus may be controlled in various ways during patient treatment.

Document WO 2012/172398 AI discloses an apparatus for extracorporeal blood treatment comprising sensors for determining a first parameter relating to a patient's blood volume, a second parameter relating to an ultrafiltration flow rate or to a patient's weight loss rate, a third parameter relating to a conductivity or concentration of a liquid crossing the dialysis line and/or the infusion line and a fourth parameter relating to an infusion flow rate. The apparatus comprises a control unit for performing a control procedure comprising: receiving, from the sensors, the measured values of the above-cited parameters and calculating, on the basis of the measured values and the prescription values of the variation in blood volume, the weight loss, the plasma conductivity or sodium concentration, the infusion volume, control values to be imposed during a time interval following the control instant.

WO 2012/172398 uses the sodium concentration in dialysis fluid to increase the extra cellular osmolality with the goal to trigger and sustain the water shift from the intracellular to the extracellular compartment (i.e. plasma refilling), just as needed.

The Applicant observed that dialysate sodium prescriptions are mostly independent of pre-dialysis serum sodium levels and this lack of individualization may lead to dialysis that ignores a sodium set point of the patient, causing unwanted deviation in terms of patient fluid balance.

The Applicant observed that the prescription values of plasma conductivity and/or sodium concentration to be achieved in the patient in the treatment time T, i.e. at the end of the extracorporeal blood treatment, is/are set by the operator but is/are not known a priori.

Therefore, the prescription value of sodium concentration is usually measured periodically (e.g. once per month) through an expensive laboratory test and adapted month by month on the base of the last laboratory data or may be estimated, treatment by treatment, relying on the doctor's knowledge of that specific patient's hystory and pathologies.

Other existing techniques to estimate the initial patient's sodium in blood and/or to target the dialysis prescription are known but they are expensives, meaning that they require dedicated disposable (eg: hemofilters) or additional work by the operator.

Document WO 2018/095694A1 is also known comprising a control unit configured for setting a sodium concentration in the dialysis fluid and after setting the dialysis fluid at the initial set point, circulating the dialysis fluid and/or the substitution fluid, measuring an initial conductivity value of the dialysate at the beginning of the treatment, and calculating, based on the measured initial conductivity value of the spent dialysis fluid and on the corresponding conductivity value of the dialysis fluid, the value of the initial plasma conductivity. The sodium concentration set point is determined based on the sodium concentration value for an isoconductive dialysis corrected based on an adjustment term to achieve an isotonic, isonatric, or isonatrikalemic dialysis.

SUMMARY

An aim of the present invention is to make available an apparatus for blood treatment which is able to improve the comfort of the patient during and after treatment.

An aim of the present invention is to provide an apparatus for blood treatment which is able to substantially maintain/achieve the correct plasma conductivity and/or sodium concentration in the blood of the patient at the end of the blood treatment.

An aim of the present invention is to provide an apparatus for blood treatment configured to set in quick, simple and reliable manner the prescription values of plasma conductivity and/or sodium concentration to be achieved in the patient at the end of the blood treatment.

An aim of the present invention is to provide an apparatus for blood treatment configured to reduce the workload of the operator and/or of the physician at the start of the treatment.

A further aim of the present invention is to make available an apparatus which is able to continuously modulate UF (or weight loss) and plasma conductivity and/or sodium concentration during the extracorporeal blood treatment, in order to get the best possible compromise between reaching the prescription targets and improving the comfort of the patient during treatment in a more physiological way.

A further aim of the present invention is to provide an apparatus which allows accelerating the search sequence for the wanted trajectories of prescription parameters and is however able to operate safely.

At least one of the above-indicated aims is substantially attained by an apparatus for blood treatment as in one or more of the appended claims.

Further characteristics and advantages of the present invention will better emerge from the detailed description that follows of at least an embodiment of the invention, illustrated by way of example in the accompanying figures of the drawings.

DESCRIPTION OF THE DRAWINGS

The invention will be described with the aid of the figures of the drawings, by way of non-limiting example, which illustrate some aspects of the invention.

In particular:

- figure 1 is a schematic illustration of an example of an extracorporeal blood treatment apparatus of the invention;

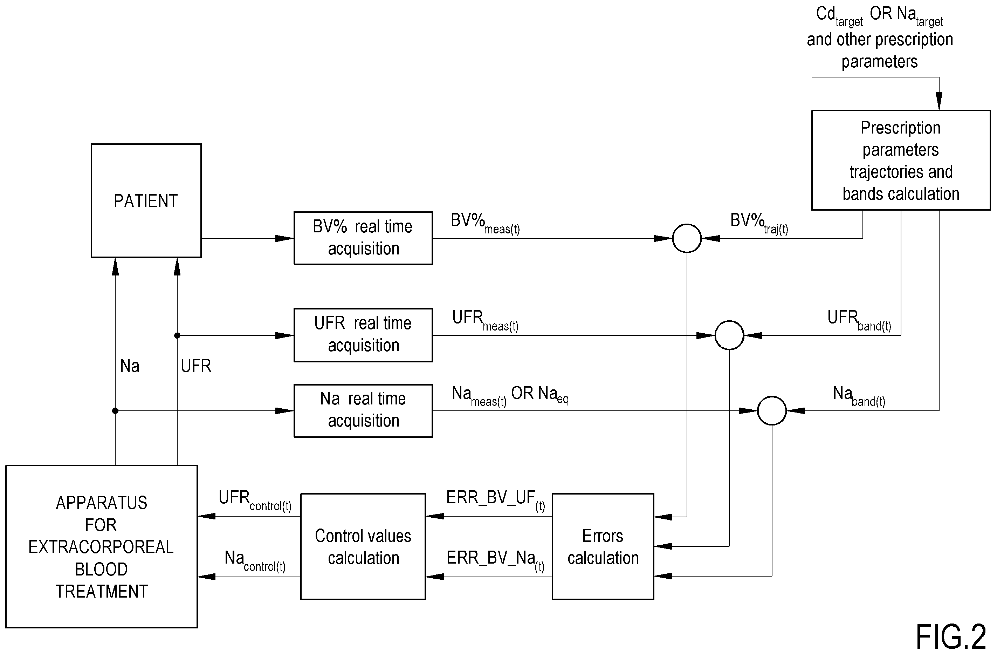

- figure 2 is a block diagram relating to an embodiment of a control procedure of the invention;
- figures 3A to 3C show the progression of parameters of the extracorporeal blood treatment controlled through the control procedure of the invention;
- figure 4 is a flow diagram relating to an embodiment of an estimating procedure of the invention;
- figure 5 is a flow diagram relating to an embodiment of a control procedure of the invention.

DETAILED DESCRIPTION

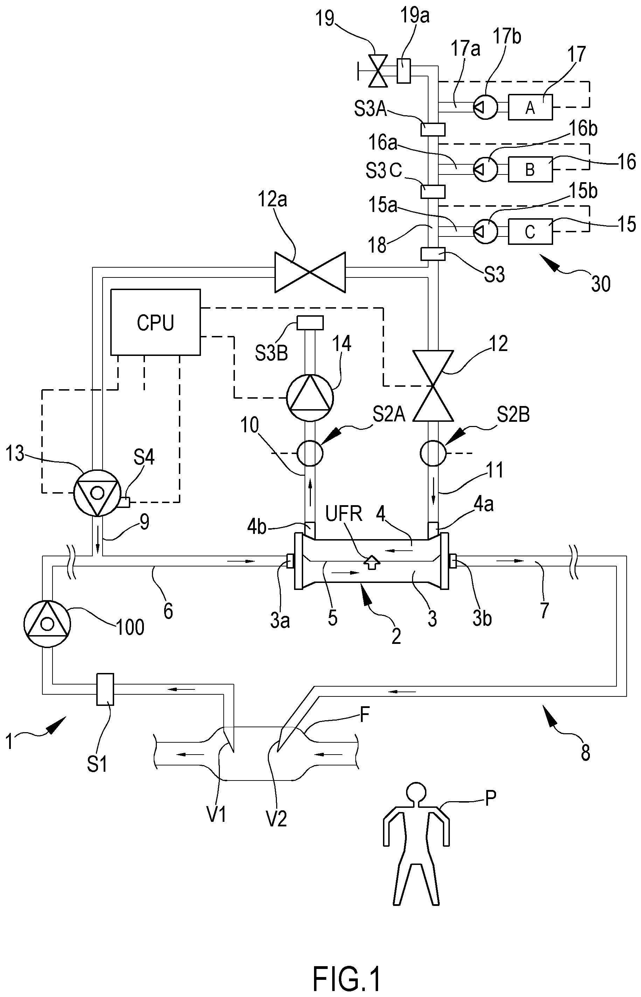

With reference to figure 1, number 1 denotes in its entirety an apparatus for extracorporeal blood treatment. The apparatus 1 comprises at least one treatment unit 2, for example a hemofilter, a hemodiafilter, a plasma filter, having at least one first chamber 3 and at least one second chamber 4 separated from one another by a semipermeable membrane 5.

A blood removal line 6 is connected with an inlet port 3a of the first chamber 3 and is predisposed, in operating conditions of connection to a patient P, to remove blood from a vascular access V1 inserted for example in a fistula F of the patient P.

A blood return line 7 connected to an outlet port 3b of the first chamber 3 is predisposed to receive the treated blood from the treatment unit 2 and to return the treated blood to a further vascular access V2 connected with the patient's fistula. Note that the configuration of the vascular access may be of any nature: for example a catheter, a port implanted in the patient, a cannula, a needle, etc. The blood removal line 6, the first chamber 3 of the treatment unit 2 and the blood return line 7 to the patient P are part of an extracorporeal blood circuit 8. A blood pump 100 on the blood removal line 6, during the use of the apparatus 1, provides for the circulation of the blood externally of the patient's body when subjected to treatment.

In the example of figure 1, an infusion line 9 of a replacement liquid is connected to the blood removal line 6, upstream of the first chamber 3. Alternatively, the infusion line 9 might be connected to the return line 7, downstream of the first chamber 3. In other examples, an infusion line may be connected downstream while another infusion line may be connected upstream of the unit 2. Further infusion lines may also be provided, for example connected downstream and/or upstream of the treatment unit 2.

The apparatus 1 further comprises at least one liquid evacuation line 10 connected with an outlet port 4b of the second chamber 4 for receiving at least a dialysate filtered across the semipermeable membrane 5. A dialysis line 11 supplies a fresh dialysis liquid to an inlet 4a of the second chamber 4. A liquid check organ 12 may be used to selectively enable or inhibit a passage of liquid across the dialysis line 11, according to whether it is desired, or not, to have a purification by diffusive effect internally of the treatment unit 2.

In the example of figure 1, the dialysis line 11 and the infusion line 9 are part of a same supply line and the dialysis liquid and the replacement fluid are the same liquid crossing the supply line.

The apparatus 1 comprises sensor devices for determining the actual values assumed during treatment by parameters described herein below.

The apparatus 1 further comprises a control unit CPU connected with the sensor devices and configured to receive prescription values of said parameters to be reached in the patient P or to follow over a treatment time (T) and to obtain, through the sensor devices, the actual values of said parameters during the extracorporeal blood treatment. The apparatus 1 further comprises a user interface, not shown, provided with input and output devices, like a keyboard, a display, a touch screen, etc. connected to the control unit CPU.

Parameters

The following parameters are considerd (prescribed and/or detected and/or adjusted) by the apparatus 1 for extracorporeal blood treatment:

- a first parameter (BV%) relating to a variation in blood volume of the patient P;
- a second parameter (UFR; WLR; WL) relating to an ultrafiltration flow rate through the semipermeable membrane 5 or to a weight loss rate of the patient P or to an accumulated weight loss;
- a third parameter (Cd, Na) relating to a conductivity of the liquid crossing the dialysis line 11 and the infusion line 9 or to a concentration of at least sodium and possible other substances (e.g. potassium, bicarbonate, calcium, magnesium, etc.) typically composing the liquid crossing the dialysis line 11 and the infusion line 9;
- a fourth parameter ($Q_{INF}$, TMP) relating to a parameter selected from: an infusion flow rate of the replacement liquid crossing the infusion line 9 or a transmembrane pressure between the first chamber 3 and the second chamber 4.

First parameter - Variation of Blood Volume

The sensor devices of the apparatus 1 comprise a first sensor S1 for detecting the actual values of the first parameter, e.g. variation of blood volume (BV%) or an actual value of a parameter from which the variation of blood volume (BV%) may be calculated in relation to the blood of a patient P subjected to treatment. The blood volume variation sensor S1 may for example be optical and able to detect a variation in the optical properties of the blood crossing a calibrated portion of tube. For instance, a blood volume variation sensor S1 may calculate, through the control unit CPU, a percentage variation of the blood volume (BV%) circulating in the patient from start of hemodialysis treatment (or hemofiltration, or hemodiafiltration) based on the measurement of the concentration of hemoglobin in the blood, according to the known formula: $BV\%(t) = (HGB_0/ HGB_t) - 1$, where $HGB_0$ represents the concentration of hemoglobin at start of treatment and $HGB_t$ the concentration of hemoglobin at time t in which variation of the blood volume (BV%) is calculated. The hemoglobin concentration is calculated based on the variation of optic absorbance, at a predetermined wavelength, of the blood flowing in the blood removal line 6, across a tract of tube having the appropriate optical properties, previously characterised.

Second parameter - Ultrafiltration flow rate

The sensor devices of the apparatus 1 comprise at least a second sensor S2A, S2B for detecting the actual values of the ultrafiltration flow rate (UFR; second parameter) across the semipermeable membrane 5. For example, a flow sensor S2A may be active on the evacuation line 10 and a flow sensor S2B on the dialysis line 11 such as to provide the control unit CPU with the instant value of the respective flows and thus enable the control unit CPU to calculate an instant ultrafiltration flow. Alternatively, a differential sensor may be provided, active on the evacuation line 10 and dialysis line 11 and therefore able directly to provide a signal relating to the ultrafiltration flow rate UFR.

The sensor or sensors S2A, S2B may be volumetric sensors, mass sensors such as for example Coriolis sensors, weight sensors such as for example scales, pump revolution sensors, or sensors of yet another type: as the type of sensors usable is not significant and since the techniques and the sensors for detecting absolute or differential flow values are known and within the experience of the expert person in the field, no further details thereof are included in the present text.

Second parameter - Weight Loss Rate

The weight loss rate (WLR; second parameter) may be measured by subtracting the infusion rate (for example as described thereafter) from the ultrafiltration flow rate (for example as described above) as $UFR= Q_{INF} + WLR$.

As a further alternative, a sensor may be provided which is able directly to provide a signal which gives the weight loss rate (WLR): for example a sensor able to differentially measure the rate taken from the evacuation line 10 and to subtract the flow rate crossing the dialysis line 11 and/or the rate or rates of infusion 9. The sensor may be a mass flow sensor (for example a Coriolis sensor), volumetric, electromagnetic, ponderal (such as a scales able to weigh bags of fluid) or another type.

Second parameter - Weight Loss

The apparatus 1 may also determine the weight loss (WL; second parameter) over a time period, for example from start of the treatment up to an instant t: for example the control unit CPU may be programmed to integrate the weight loss rate (WLR) over the time. Alternatively, a weight loss sensor may be provided, for example a sensor configured to detect the variation in overall weight of a patient P during treatment or a sensor destined to directly detect the overall weight of the net fluid extracted from the patient P.

Third parameter - Conductivity or sodium concentration

The sensor devices of the apparatus 1 further comprise at least a third sensor S3 for detecting the actual values of the third parameter, i.e. conductivity or concentration of at least sodium (plus another substance, like potassium) that is to be monitored of the liquid crossing the dialysis line 11 and/or the infusion line 9. For instance, the conductivity or concentration sensor S3 may be located immediately downstream of a device for regulating a composition of dialysis liquid and/or replacement fluid, which will be more fully described in the following.

The sensor devices of the apparatus 1 further comprise a third sensor S3B for detecting the actual values of the third parameter, i.e. conductivity or concentration of at least sodium, in the dialysate downstream of the second chamber 4 which is located on the liquid evacuation line 10.

The apparatus 1 may also not comprise a conductivity or concentration sensor directly acting on the patient or on the extracorporeal blood circuit. In this case, the control procedure uses a mathematical model M representing a kinetics of solutes in a distribution volume V in the patient for iteratively calculating, at each control instant t, an equivalent sodium concentration value $Na_{eq(t)}$.

Note that by equivalent sodium concentration at instant t ($Na_{eq(t)}$) reference is made to the constant sodium concentration in the dialysis liquid that, if applied at the start of treatment up to an instant t, would lead to the same plasma sodium concentration in the patient as is obtained at the same instant t with the variation of sodium concentration or conductivity set by the control procedure up to time t. In this case, also the prescription values of sodium concentration are prescription values of equivalent sodium concentration.

Fourth parameter - Infusion flow rate

The sensor devices of the apparatus 1 further comprise at least a fourth sensor S4 for detecting the actual values of the infusion flow rate ($Q_{INF}$; fourth parameter) of the replacement liquid crossing the infusion line 9. The fourth sensor or sensors S4 may be volumetric sensor/s, mass sensor/s such as for example Coriolis sensor/s, weight sensor/s such as for example scales, pump revolution sensor/s or sensor/s of still other types: as the type of sensors usable is not significant and since the techniques and the sensors for detecting absolute or differential flow values are known and within the experience of the expert person in the field, no further details thereof are included in the present text. In the case illustrated in figure 1, the infusion flow rate sensor S4 is configured to determine a number of revolutions of an infusion pump 13, by sending a corresponding signal to the control unit CPU which is configured such as to calculate a flow rate along the respective infusion line 9.

Regulating device

The apparatus 1 comprises a regulating device for regulating the second parameter. The regulating device are connected to the control unit CPU and are active on at least one of the extracorporeal blood circuit 8 and the liquid evacuation line 10. In the example of figure 1, the regulating device comprises an ultrafiltration pump 14 located on the liquid evacuation line 10 and able to recall fluid from the second chamber 4. In other embodiments, the regulating device may comprise two pumps piloted differentially, such as two blood pumps located one upstream and one downstream of the treatment unit 2, or a plurality of pumps located on the lines and piloted such as to create an ultrafiltration flow across the membrane 5 or combinations of one or more pumps and valves appropriately arranged on the blood lines 6, 7 or liquid evacuation line 10. The control unit CPU is connected to the regulating device to pilot the pump or pumps such as to adjust the ultrafiltration flow rate (UFR) as will be detailed thereafter.

Liquid regulating device

The apparatus 1 further comprises a liquid regulating device 30 for regulating the third parameter, i.e. the composition of the liquid crossing the supply line, i.e. of the dialysis liquid and/or of the replacement fluid.

In the example of figure 1, the liquid regulating device 30 comprises one, two or more containers of concentrate 15, 16, 17 located on respective injection lines 15a, 16a, 17a which are predisposed to supply substances such as electrolytes, buffer agents or others towards a preparation line 18 of the liquid located upstream of the supply line.

The concentrate containers 15, 16, 17 may comprise concentrates in the liquid state or solid state, for example powder. Injection pumps 15b, 16b, 17b may be present on the injection lines 15a, 16a, 17a to move the fluid along the respective injection line towards the preparation line 18 which collects the liquid, for example water, from a source 19. The source 19 may comprise a deionized water source or a source of ultra-pure liquid. The water collected from the source and possibly subjected to filtering stages 19a (not detailed as known and not relevant to the present invention) is provided with the required substances.

According to the embodiment shown in figure 1, the supply line comprises the infusion line 9 and the dialysis line 11 which depart both from the preparation line 18. Therefore, the dialysis fluid and the replacement fluid are the same liquid prepared by the preparation line 18 liquid and crossing the supply line. A further fluid check organ 12a may also be used to selectively enable or inhibit a passage of fluid across the infusion line 9.

The concentration or conductivity sensor S3, possibly added-to by further concentration or conductivity sensors S3A, S3C located on the preparation line 18, is able to provide the control unit CPU with a relative signal of conductivity or concentration of a predetermined substance (like sodium and/or potassium) of the fluid crossing the preparation line 18 such that the control unit CPU may act on the liquid regulating device and in particular on the pumps 15b, 16b, 17b in order to regulate the conductivity (Cd) or concentration, for example of sodium (Na) and/or potassium (K), of the liquid crossing the dialysis line 11 and/or the infusion line 9.

The infusion line 9 may also collect the fluid from a further source (for example a bag containing replacement fluid, not shown) independent with respect to the water source 19, while the preparation line 18 exclusively supplies the dialysis line 11.

Procedures

The control unit CPU may comprise one or more digital units, for example microprocessors, or one or more analog units, or a special combination of digital and analog units. The control unit CPU is connected with the regulating devices, with the user interface, with the sensor devices and with the various actuator organs (blood pump 100, infusion pump 13, ultrafiltration pump 14, fluid check organs 12, 12A) located along the lines 6, 9, 10, 11 and is configured or programmed to perform the procedures described herein. In a case in which the control unit CPU is programmable, the control unit CPU is connected with a data support for storing instructions which, when performed by the control unit CPU, determine performing of the procedures which will be described herein below. The data support may comprise a mass data memory, for example optical or magnetic, a re-programmable memory (EPROM, FLASH) or a memory of another nature. In an aspect of the invention, the control unit CPU is programmed or configured such as to perform a "Procedure for estimating targets" and a "Control procedure" comprising the steps described herein below.

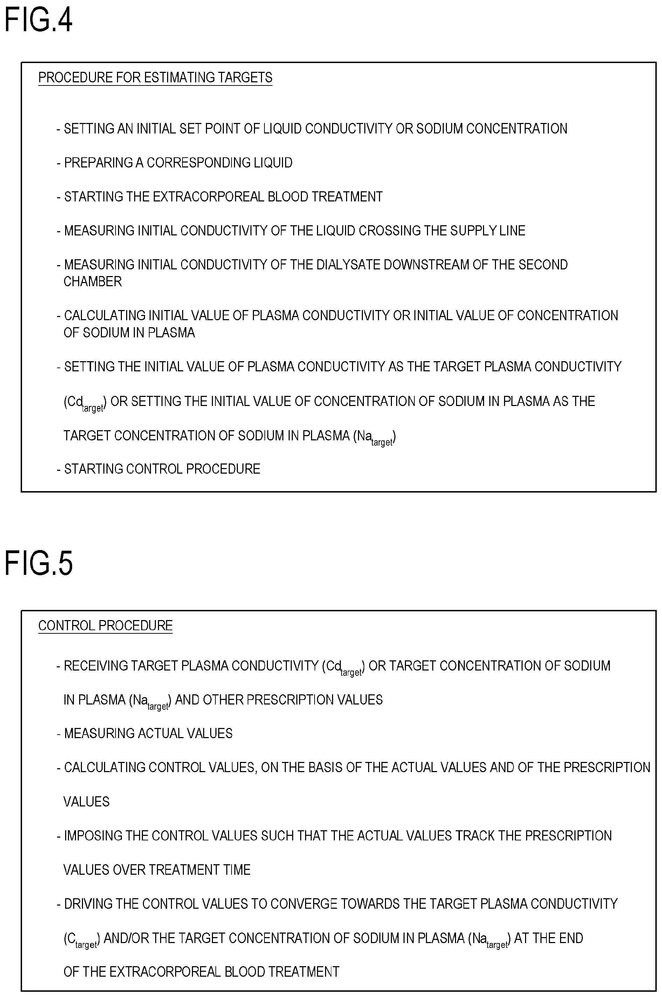

Control procedure (Figure 5)

According to an example of the control procedure, in a first step, the control unit CPU receives, for instance via the user interface:

- a treatment time (T),
- an estimated or measured initial concentration of haemoglobin and/or an initial blood volume ($BV_{init}$) of the patient;
- a target variation in blood volume ($BV\%_{target}$) to be reached at the end of the blood treatment;
- a target ultrafiltration volume ($UF_{target}$) or target weight loss ($WL_{target}$) to be reached at the end of the blood treatment;
- an initial set point of conductivity or sodium concentration ($C_{dinit}$, $Na_{init}$) of the liquid crossing the supply line 11;
- a target plasma conductivity and/or a target concentration of sodium in plasma ($Cd_{target}$, $Na_{target}$) to be reached in the patient P at the end of the blood treatment.

The control unit CPU calculates a first parameter trajectory ($BV\%_{traj(t)}$) from the estimated or measured initial concentration of haemoglobin and/or an initial blood volume of the patient P, the target variation in blood volume ($BV\%_{target}$) and the treatment time (T). The control unit CPU also calculates a first parameter band delimited between an upper trajectory (above the first parameter trajectory ($BV\%\ t_{raj(t)}$)) and a lower trajectory (below the first parameter trajectory ($BV\%\ t_{raj(t)}$)), wherein a mid line of the first parameter band defines said first parameter trajectory ($BV\%_{traj(t)}$).

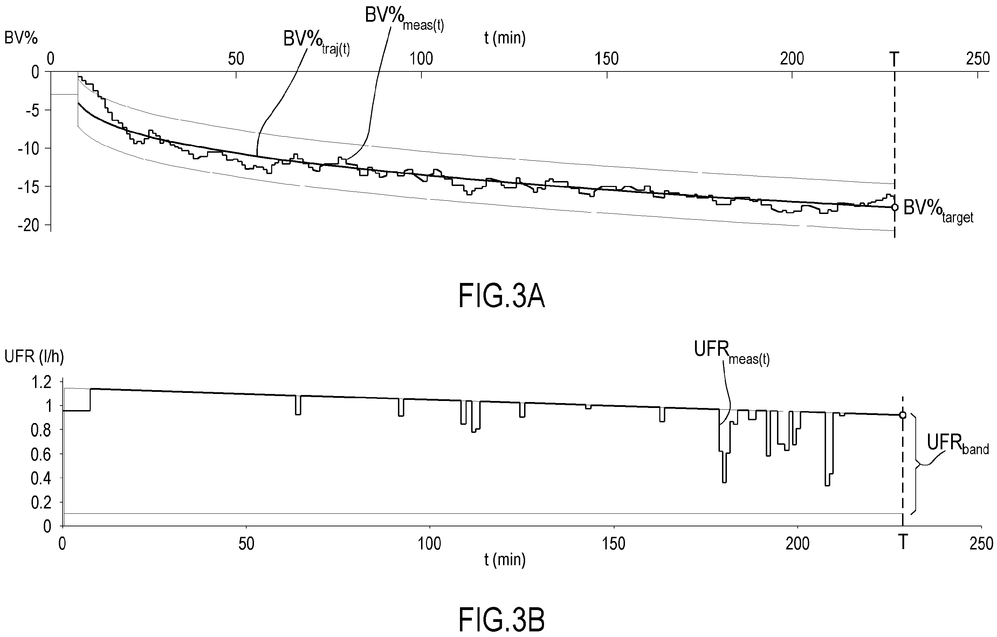

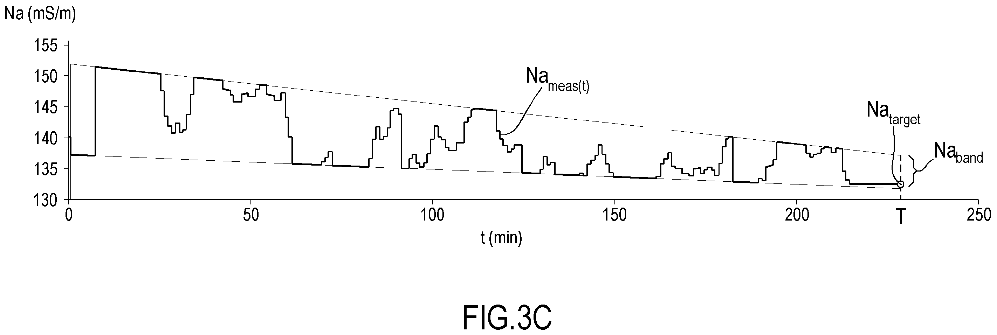

As shown in figure 3A, the first parameter trajectory ($BV\%_{traj(t)}$) comprises a succession of prescription values of the variation in blood volume (BV%) to be followed over the treatment time. Usually, the first parameter trajectory ($BV\%_{traj(t)}$) decreases over treatment time. The mid line of the fourth parameter band shown in figure 3A is in the middle, i.e. equally spaced from the upper trajectory and lower trajectory, but said mid line may also be eccentric compared to the upper trajectory and lower trajectory. The control unit CPU calculates, from the target ultrafiltration volume ($UF_{target}$) or target weight loss ($WL_{target}$) and the treatment time (T), a second parameter band or ultrafiltration flow rate band ($UFR_{band}$) delimited between an upper trajectory and a lower trajectory over the treatment time. Usually, the upper trajectory and a lower trajectory are straight lines slightly decreasing and converging one towards the other over treatment time, like in figure 3B. The points of the the upper trajectory and the lower trajectory and comprised between the upper trajectory and the lower trajectory are prescription values of the UFR. The control unit CPU calculates, from the initial set point of conductivity or sodium concentration ($C_{dinit}$, $Na_{init}$), from the target plasma conductivity and/or the target concentration of sodium in plasma ($Cd_{target}$, $Na_{target}$) and the treatment time (T), a third parameter band or conductivity or sodium concentration band ($Cd_{band}$, $Na_{band}$) delimited between an upper trajectory and a lower trajectory over the treatment time. Usually, the upper trajectory and the lower trajectory are straight lines slightly decreasing and converging one towards the other over the treatment time, like in figure 3C. The points of the the upper trajectory and the lower trajectory and comprised between the upper trajectory and the lower trajectory are prescription values of the Cd or Na.

Blood treatment is started and the control unit CPU receives continuously and in real time during treatment (real time acquisition):

- the actual values of the variation of blood volume ($BV\%_{meas(t)}$) from the first sensor S1 (figure 3A);
- the actual values of the ultrafiltration flow rate ($UFR_{meas(t)}$) from the second sensors S2A, S2B (figure 3B);
- the actual values of the conductivity or sodium concentration ($Cd_{meas(t)}$, $Na_{meas(t)}$) in the liquid crossing the supply line 11 from the third sensor S3 (figure 3C).

As remarked before, the actual values of the sodium concentration may be values of equivalent sodium concentration value $Na_{eq(t)}$ iteratively calculated as disclosed above.

The control unit CPU calculates, at temporally consecutive control instants and on the basis of the actual values and of the prescription values, the following control values to be set during a time interval after the instant in which the control is made:

- a second parameter control value. i.e. ultrafiltration flow rate control value ($UFR_{control(t)}$); and/or
- a third parameter control value, i.e. conductivity or sodium concentration control value ($Cd_{control(t)}$; $Na_{control(t)}$).

The control unit CPU commands the ultrafiltration pump 14 (regulating device) to impose the ultrafiltration flow rate control value ($UFR_{control(t)}$) during a time interval consecutive to the instant in which the control is made. In other words, the ultrafiltration pump 14 is adjusted such that the actual value of the ultrafiltration flow rate $UFR_{meas(t+\Delta t)}$ at the time interval after the instant in which the control is made is equal or close to the ultrafiltration flow rate control value ($UFR_{control(t)}$). The control unit CPU commands the injection pumps 15b, 16b, 17b (liquid regulating device) to impose the conductivity or sodium concentration control value ($Cd_{control(t)}$; $Na_{control(t)}$) during a time interval consecutive to the instant in which the control is made. In other words, the injection pumps 15b, 16b, 17b are adjusted such that the actual values of the conductivity or sodium concentration ($Cd_{meas(t+\Delta t)}$, $Na_{meas(t+\Delta t)}$) at the time interval after the instant in which the control is made is equal or close to the conductivity or sodium concentration control value ($Cd_{control(t)}$; $Na_{control(t)}$).

The ultrafiltration flow rate control value ($UFR_{control(t)}$) and the the conductivity or sodium concentration control value ($Cd_{control(t)}$; $Na_{control(t)}$) are calculated and imposed such that the actual values of the variation of blood volume ($BV\%_{meas(t)}$) track the prescription values of the variation of blood volume (first parameter trajectory $BV\%_{traj(t)}$) over the treatment time (T). For instance, the ultrafiltration flow rate control value ($UFR_{control(t)}$) and the the conductivity or sodium concentration control value ($Cd_{control(t)}$; $Na_{control(t)}$) are calculated and imposed such that the values of the variation of blood volume ($BV\%_{meas(t)}$) follows the prescribed trajectory or is kept in a neighborough of said the prescribed trajectory or within the prescribed band ($BV\%_{traj(t)}$, figure 4A). In addition, the ultrafiltration flow rate control value ($UFR_{control(t)}$; $WLR_{control(t)}$; $WL_{control(t)}$) is calculated to be within the ultrafiltration flow rate band ($UFR_{band}$) and the conductivity or sodium concentration control value is calculated to be within the conductivity or sodium concentration band ($Cd_{band}$, $Na_{band}$), i.e. to keep the respective actual values within the respective bands, as shown in figures 3B and 3C. The ultrafiltration flow rate control value ($UFR_{control(t)}$; $WLR_{control(t)}$; $WL_{control(t)}$) and/or the conductivity or sodium concentration control value ($Cd_{control(t)}$; $Na_{control(t)}$) may also be null, i.e. the control unit CPU may stop ultrafiltration and/or sodium administration.

The control values ($UFR_{control(t)}$; $Cd_{control(t)}$; $Na_{control(t)}$) may be calculated as follows.

The control unit CPU determines a first error parameter ($ERR\_BV\_UF_{(t)}$) on the basis of a difference between the actual value of the variation of blood volume ($BV\%_{meas(t)}$) at the control instant (t) and a corresponding value on the first parameter trajectory ($BV\%_{traj(t)}$) and a difference between an actual value of the second parameter ($UFR_{meas(t)}$; $WLR_{meas(t)}$; $WL_{meas(t)}$) at the control instant (t) and a corresponding value of the second parameter band. The control unit CPU determines a second error parameter ($ERR\_BV\_Na(t)$) on the basis of a difference between the actual value of the variation of blood volume ($BV\%_{meas(t)}$) at the control instant (t) and a corresponding value on the first parameter trajectory ($BV\%_{traj(t)}$) and a difference between an actual value of the third parameter ($Cd_{meas(t)}$; $Na_{meas(t)}$) at the control instant (t) and a corresponding value of the third parameter band. Then, the control unit CPU calculates a first value of the second parameter control value ($UFR_{control(t)}$; $WLR_{control(t)}$; $WL_{control(t)}$) on the basis of the first error parameter ($ERR\_BV\%\_UF_{(t)}$ and of the actual value of the second parameter ($UFR_{meas(t-\Delta t)}$; $WLR_{meas(t-\Delta t)}$; $WL_{meas(t-\Delta t)}$)) relating to a preceding control instant and calculates a first value of the third parameter control value ($Cd_{control(t)}$; $Na_{control(t)}$) on the basis of the second error parameter (ERR_BV%_Na$_{(t)}$ and of the actual value of the third parameter (Cd$_{meas(t-\Delta t)}$, Na$_{meas(t-\Delta t)}$) relating to a preceding control instant. Near the end of the extracorporeal blood treatment, the control procedure comprises: driving the third parameter control values to converge towards the target plasma conductivity and/or the target concentration of at least sodium in plasma such that at the end of the extracorporeal blood treatment the third parameter control value is close or substantially equal to the target plasma conductivity (Cd$_{target}$) and/or the target concentration of sodium in plasma (Na$_{target}$).

The control procedure above described is automatically managed by the control unit CPU. In case, despite the automatic control procedure, the actual values of the variation of blood volume (BV%$_{meas(t)}$) move away from the respective prescription values (move away from a prescribed trajectory or move outside a prescribed band or move above or below a prescribed threshold), the control unit CPU is programmed to issue an alarm signal to alert the staff and/or to stop the blood treatment.

Procedure for estimating targets (Figure 4)

In an aspect of the invention, the control unit CPU is programmed or configured such as to perform, at least at the beginning of the extracorporeal blood treatment, an estimation procedure for automatically estimating/calculating said target plasma conductivity and/or said target concentration of sodium in plasma (Cd$_{target}$, Na$_{target}$) to be reached in the patient at the end of the blood treatment. The control procedure described above starts when the procedure for estimating is completed.

The procedure for estimating comprises the steps described herein below.

First, the control unit CPU sets the initial set point of conductivity or sodium concentration (C$_{dinit}$, Na$_{init}$) of the liquid crossing the supply line 11. The set point is calculated before starting the blood circulation (i.e. before starting the extracorporeal blood treatment). In detail, the control unit CPU is configured to set the parameter value for the liquid at the initial set point so that a liquid conductivity matches a first estimate of the plasma conductivity of the blood and may be calculated as a function of the type of extracorporeal blood treatment mode, i.e. hemodialysis (HD), hemofiltration (HF) or hemodiafiltration (HDF).

The initial set point of sodium concentration (Na$_{init}$) of the liquid crossing the supply line 11 may be calculated as a function of: a concentration of one or more further substances (in addition to sodium) in the liquid crossing the supply line, an estimated plasma concentration of one or more substances, molar conductivities of one or more substances in the liquid crossing the supply line, at least one flow rate, as the dialysate flow rate at the outlet of the second chamber, an efficiency parameter of the treatment unit, like an urea clearance of the treatment unit. For instance, in hemodialysis (HD) mode, the control unit CPU is configured to calculate the initial set point of sodium concentration to be set in the liquid crossing the supply line before the start of the treatment using the following relationship:

(Equation 1)

$$c_{di,Na,start} = \alpha * c_{pw,Na} + \frac{1}{M_{\kappa_{NaCl}}}(M_{\kappa_{NaHCO_3}} - M_{\kappa_{NaCl}})(\frac{1}{\alpha} * c_{pw,HCO_3} - c_{di,HCO_3}) +$$

$$+ \frac{1}{M_{\kappa_{NaCl}}}(M_{\kappa_{NaAc}} - M_{\kappa_{NaCl}})(\frac{1}{\alpha} * c_{pw,Ac} - c_{di,Ac}) + \frac{M_{\kappa_{KCl}}}{M_{\kappa_{NaCl}}}(\alpha * c_{pw,K} - c_{di,K}) +$$

$$+ \frac{1}{M_{\kappa_{NaCl}}} \frac{Q_{do}}{K_u} \kappa_{rest3}$$

wherein the used symbols meaning is clarified in the following table 1.

Table 1

| Name | Description | Unit |
|---|---|---|
| $Q_{do}$ | Dialysate flow rate at the outlet of the second chamber | mL/min |
| $K_u$ | Treatment unit clearance for urea | mL/min |
| $C_{di.K}$ | Concentration of potassium ions in the liquid crossing the supply line as determined by the used concentrate | mmol/L |

(continued)

| Name | Description | Unit |
|---|---|---|
| $C_{di.Ac}$ | Concentration of acetate in the liquid crossing the supply line as determined by the used concentrate | mmol/L |
| $C_{vw.Na}$ | Estimated or measured pre-dialysis concentration of sodium ions in plasma water | mmol/L |
| $C_{vw.HCO}$ | Estimated or measured pre-dialysis concentration of bicarbonate anions in plasma water | mmol/L |
| $C_{vw.Ac}$ | Estimated or measured pre-dialysis concentration of acetate anions in plasma water | mmol/L |
| $C_{vw.K}$ | Estimated or measured pre-dialysis concentration of potassium ions in plasma water | mmol/L |
| $k_{rest3}$ | Conductivity contribution from lesser solutes 3 | mS/cm |
| $M_{\kappa.NaHCO}$ | Molar conductivity of sodium bicarbonate at ionic strength 150 mM | L·mS/mol·cm |
| $M_{\kappa.NaCl}$ | Molar conductivity of sodium chloride at ionic strength 150 mM | L·mS/mol·cm |
| $M_{\kappa.NaAc}$ | Molar conductivity of sodium acetate at ionic strength 150 mM | L·mS/mol·cm |
| $M_{\kappa.KCl}$ | Molar conductivity of potassium chloride at ionic strength 150 mM | L·mS/mol·cm |
| $\alpha$ | Donnan factor | |

In case of HDF post-dilution mode is selected, the same equation (1) applies. Differently from HD mode, treatment unit clearance $K_u$ is calculated for the HDF post-dilution mode. In case of HDF pre-dilution mode is selected, the dilution of the blood before it enters the treatment unit should be taken into account. The equations for HF post-dilution mode are the same as for to HD, but the treatment unit clearance is substituted by the dialysate flow rate at treatment unit outlet, i.e. $K_u=Q_{do}$. In case of HF pre-dilution mode is selected, the dilution of the blood before it enters the dialyzer should be taken into account.

Once the sodium initial set point has been calculated, a corresponding liquid is prepared by the control unit CPU driving the preparation line 18. Then the extracorporeal blood treatment is started by circulating the liquid through the supply line and by circulating blood through the extracorporeal blood circuit 8.

Depending on the selected treatment mode, the dialysis fluid is directed:

- to the second chamber 4 of the treatment unit 2 only so as to exchange with blood (HD mode);
- to the second chamber 4 of the treatment unit 2 so as to exchange with blood and infused into the blood circuit 8 (HDF mode);
- to the blood circuit 8 only (HF mode).

Correspondingly, blood is withdrawn from the patient P and circulated in the extracorporeal blood circuit 8 and particularly is circulated through the first chamber 3 of the filtration unit 2.

Then at least one, and in general a plurality, of consecutive initial values of the third parameter (in the example, the conductivity) of the dialysate downstream of the second chamber 4 are measured at the beginning of the treatment through the third sensor S3B.

The dialysate conductivity will change initially, due to e.g. dynamics when treatment is started (e.g. leaving bypass conditions) or when blood flow is ramped up. However, it is expected to stabilize within few (e.g. 4) minutes. The measurement is made as soon as stability criteria are fulfilled. The control unit CPU is configured to validate and further process the measurement of an initial value of the conductivity of the dialysate as soon as the diffusion process in the treament unit 2 reaches stable conditions.

In order to minimize the time needed to reach stability conditions, changes in the flow rate of liquid crossing the supply line and in bicarbonate prescription may be prevented during this preliminary sodium identification phase. Changes in blood flow, ultrafiltration flow rate or bypass are vice versa generally allowed, but they will delay stability. Moreover, it is not possible to change the concentrate combination type after the treatment is started.

An initial conductivity of the liquid crossing the supply line upstream the second chamber 4 is either measured or taken as the set value for liquid conductivity. In general, the initial conductivity of the liquid may be measured through the third sensor S3.

The initial setting of the sodium concentration calculated or determined as above stated to be as close as possible to the expected plasma conductivity (equation 1) may be optional, meaning that the method for estimating the initial plasma conductivity may be performed even if the sodium content of the sodium concentration to be set in the liquid crossing the supply line is initially simply set by the operator.

The control unit CPU calculates an initial value of plasma conductivity based on the measured initial parameter value of the dialysate (i.e. based on conductivity or concentration measurement of dialysate on the treatment unit outlet) and on the corresponding parameter value of the liquid crossing the supply line (e.g. conductivity or concentration). During the start of the treatment and particularly during circulating the liquid through the second chamber 4 and/or in the infusion line 9 up to measuring the initial value of the parameter of the dialysate downstream of the second chamber 4 used for the calculating of the initial plasma conductivity, the liquid conductivity (or concentration of sodium) is kept substantially constant. In other words, the calculation of the initial plasma conductivity is performed with no conductivity step.

After calculating the initial value of plasma conductivity, the control unit CPU is configured to drive a liquid regulating device 30 to change the composition of the liquid crossing the supply line to reach a liquid conductivity substantially equal to the initial value of plasma conductivity (isoconductivity).

According to a first embodiment, the control unit CPU is programmed to calculate the initial value of plasma conductivity based on the sum of at least the initial conductivity of the dialysate plus a difference between inlet and outlet conductivity at the treatment unit 2, or dialyzer, weighted by a factor of the dialysate flow rate. In more detail, the difference between inlet and outlet conductivity at the treatment unit 2 is weighted by a factor of the blood flow rate in the blood lines too.

According to the first embodiment, the control unit CPU is configured to calculate the initial value of plasma conductivity using the following formula:

(Equation 2)

$$\kappa_{p,1}' = \kappa_{0,do} + \frac{Q_{do}}{Q_{Bset}}\left(\kappa_{0,do} - \kappa_{0,di}\right)$$

The significance of the denotations above is given in the following table 2.

Table 2

| | |
|---|---|
| $\kappa_{p,1}$ | plasma conductivity first estimate; |
| $Q_{do}$ | dialysate flow rate at the treatment unit outlet; |
| $Q_{bset}$ | set blood flow rate at the treatment unit inlet; |
| $\kappa_{0,di}$ | liquid conductivity at the treatment unit inlet for a pure electrolyte solution; |
| $\kappa_{0,do}$ | dialysate conductivity at the treatment unit outlet for a pure electrolyte solution. |

It is worth to underline that during the above described calculation of the initial plasma conductivity (Equation 2), the liquid circulates through the second chamber 4 and/or is infused into the blood circuit 8 (depending on selected HD/HF/HDF mode) maintaining the liquid parameter value substantially constant.

In a second embodiment, the control unit CPU is programmed to calculate the initial plasma conductivity based on the sum of at least the initial conductivity of the fresh liquid plus a difference between inlet and outlet conductivity at the treatment unit 2 weighted by a factor of the dialysate flow rate. In more detail the difference between inlet and outlet conductivity at the treatment unit 2, or dialyzer, is weighted by a factor of the dialyzer clearance too.

According to the second embodiment, the control unit CPU is configured to calculate the plasma conductivity using the following formula:

(Equation 3)

$$\kappa_{p,1}'' = \kappa_{0,di} + \frac{Q_{do}}{K_u}\left(\kappa_{0,do} - \kappa_{0,di}\right)$$

The significance of the denotations above is given in the following table 3.

Table 3

| $\kappa_{p,1}$ | plasma conductivity first estimate; |
|---|---|
| $Q_{do}$ | dialysate fluid flow rate at the treatment unit outlet; |
| $K_u$ | treatment unit clearance for urea; |
| $_{0,di}$ | liquid conductivity at the treatment unit inlet for a pure electrolyte solution; |
| $_{0,do}$ | dialysate conductivity at the treatment unit outlet for a pure electrolyte solution. |

Of course, both formulas (2) and (3) for estimation of plasma conductivity may be iteratively applied, meaning that the newly calculated estimate of plasma conductivity ($k_{p,1}$) is imposed to the liquid (through the liquid regulating device 30) and a new estimate ($k_{p,2}$) again calculated after taking measures of the conductivity at the inlet and outlet of the treatment unit 2 as soon as stable conditions are reached.

Once the initial value of plasma conductivity has been estimated through the "Procedure for estimating targets", said initial value of plasma conductivity is set or imposed as the target plasma conductivity ($Cd_{target}$) to be reached in the patient P at the end of the extracorporeal blood treatment and used in the "Control procedure" formerly disclosed.

According to a different embodiment, once the initial value of plasma conductivity has been estimated through the "Procedure for estimating targets", the initial value of concentration of sodium in plasma is derived from the initial value of plasma conductivity and said initial value of concentration of sodium in plasma is set or imposed as the target concentration of sodium in plasma ($Na_{target}$) to be reached in the patient P at the end of the extracorporeal blood treatment and used in the "Control procedure" formerly disclosed.

Said initial value of concentration of sodium in plasma (or target concentration of sodium in plasma $Na_{target}$) may be set as the concentration of sodium in the liquid crossing the supply line when the liquid conductivity matches the initial value of plasma conductivity (or target plasma conductivity $Cd_{target}$), i.e. when the liquid tonicity is not changed during its passage through the treatment unit 2 and said liquid tonicity matches the tonicity of plasma.

According to other embodiments, adjustment factors are applied to the estimated initial value of plasma conductivity or estimated initial value of concentration of sodium in plasma to obtain corrected values of plasma conductivity or concentration of sodium in plasma and then the target plasma conductivity ($Cd_{target}$) or the target concentration of sodium in plasma ($Na_{target}$).

These adjustment factors may be chosen by a doctor. In other words, the estimation of the initial patient's plasma sodium concentration/conductivity may be used as reference for the doctor to eventually decide to correct the target final patient's plasma sodium concentration/conductivity.

These adjustment factors may also be calculated. In this instance, the target final patient's plasma sodium concentration/conductivity is the sum of a main contribution term based on/function of the plasma conductivity and as a function of the adjustment factor. According to an ambodiment, the target concentration of sodium in plasma is set as the sum of the concentration of sodium in the liquid crossing the supply line when the liquid conductivity matches the initial value of plasma conductivity (main contribution term) and of a concentration adjustment factor, according to the following general formula:

(Equation 4)

$$c_{di,Na,set} = c_{di,Na,\kappa_{p,pre}} + c_{di,Na,adj}$$

wherein

$c_{di,Na,\kappa}$ is the main contribution term of the liquid crossing the dialysis line at isocondictive state; and

$c_{di,N\alpha,adj}$ is the sodium concentration set point adjustment relative to an isoconductive state, which would provide a treatment which may be, for example, chosen in the group including isotonic dialysis, isonatric dialysis, and isonatrikalemic dialysis.

The main contribution term usually affects the initial value of concentration of sodium in plasma for 80% - 90% of the initial value of concentration of sodium in plasma while the concentration adjustment factor contributes to the initial value of concentration of sodium in plasma for 10% - 15%, of the initial value of concentration of sodium in plasma.

Even if the initial plasma sodium concentration/conductivity and the final/target plasma sodium concentration/-

conductivity ($Cd_{target}$, $Na_{target}$) are known/set thorugh the "Procedure for estimating", during the extracorporeal blood treatment, the values of the actual sodium concentration/conductivy of the liquid and of plasma continuously change according to the "Control procedure". Only at the end of the extracorporeal blood treatment, the control unit CPU drives the liquid regulating device 30 to change the composition of the liquid crossing the supply line to reach the target concentration of sodium in plasma $Na_{target}$ or the target plasma conductivity $Cd_{target}$.

During the extracorporeal blood treatment, the control unit may also be configured to check the conductivity of plasma in patient and/or the concentration of plasma sodium in patient to monitor treatment evolution. Values of plasma conductivity and/or of concentration of sodium in plasma may be calculated through a procedure similar to the procedure for estimating targets disclosed above.

For instance, the control unit CPU calculates the plasma conductivity from measured values of conductivity in the liquid evacuation line and in the supply line and through Equations 2 or 3 above outlined.

**Claims**

1. An apparatus for extracorporeal blood treatment, comprising:

> a treatment unit (2) having a first chamber (3) and a second chamber (4) separated from one another by a semipermeable membrane (5);
> a blood removal line (6) connected to an inlet port (3a) of the first chamber (3) and predisposed to remove blood from a patient (P),
> a blood return line (7) connected to an outlet port (3b) of the first chamber (3) and predisposed to return treated blood to the patient (P); the blood removal line (6), the blood return line (7) and the first chamber (3) being part of an extracorporeal blood circuit (8);
> at least a liquid evacuation line (10) connected to an outlet port (4b) of the second chamber (4);
> a supply line (9, 11) connected to an inlet port (4a) of the second chamber (4) and/or connected to the extracorporeal blood circuit (8);
> sensor devices for detecting actual values of parameters of the extracorporeal blood treatment;
> a control unit (15) connected with the sensor devices and configured to receive prescription values of said parameters to be reached in the patient (P) or to follow over a treatment time (T) and to obtain, through the sensor devices, the actual values of said parameters during the extracorporeal blood treatment; wherein said parameters comprises:
>
>> - a first parameter (BV%) relating to a variation in blood volume of the patient (P);
>> - a second parameter (UFR; WLR; WL) relating to an ultrafiltration flow rate through the semipermeable membrane (5) or to a weight loss rate of the patient (P) or to an accumulated weight loss;
>> - a third parameter (Cd, Na) chosen in a group consisting of: conductivity of a liquid crossing the supply line (9, 11), a conductivity-related parameter of the liquid crossing the supply line (9, 11), concentration of at least sodium in the liquid crossing the supply line (9, 11) and a concentration-related parameter of at least sodium in the liquid crossing the supply line (9, 11);
>
> wherein the control unit (15) is configured to perform, at temporally consecutive control instants (t), a control procedure comprising:
>
>> - calculating, on the basis of the actual values and of the prescription values, the following control values to be set during a time interval ($\Delta$t) after the instant (t) in which the control is made:
>>
>>> a second parameter control value ($UFR_{control(t)}$; $WLR_{control(t)}$, $WL_{control(t)}$); and
>>> a third parameter control value ($Cd_{control(t)}$; $Na_{control(t)}$);
>>
>> - imposing at least one of the second parameter control value ($UFR_{control(t)}$; $WLR_{control(t)}$, $WL_{control(t)}$) and third parameter control value ($Cd_{control(t)}$; $Na_{control(t)}$) during the time interval ($\Delta$t) consecutive to the instant (t) in which the control is made such that the actual values of the first parameter ($BV\%_{meas(t)}$) track the prescription values of the first parameter ($BV\%_{traj(t)}$) over the treatment time (T);
>
> wherein the prescription value of the third parameter (Cd, Na) comprises a target plasma conductivity ($C_{target}$) and/or a target concentration of at least sodium in plasma ($Na_{target}$) to be reached in the patient (P) in the treatment time (T); and **characterized in that** the control unit (15) is further configured to perform, at least at the beginning of the extracorporeal blood treatment, a procedure for estimating said target plasma conductivity ($C_{target}$) and/or said target concentration of at least sodium in plasma ($Na_{target}$).

2. The apparatus of claim 1, wherein at least one time during the extracorporeal blood treatment, the control unit is configured to check plasma conductivity and/or concentration of at least sodium in plasma.

3. The apparatus of claim 1 or 2, comprising a preparation line (18) of the liquid crossing the supply line (9, 11) located upstream of and connected to the supply line (9, 11); wherein the procedure for estimating comprises:

   - setting an initial set point of the third parameter of the liquid crossing the supply line (9, 11) and preparing a corresponding liquid through the preparation line (18);
   - after setting the initial set point, starting extracorporeal blood treatment by circulating the liquid through the supply line (9, 11) and by circulating blood through the extracorporeal blood circuit (8);
   - measuring an initial value of the third parameter (Cd, Na) of the dialysate in the liquid evacuation line (10) at the beginning of the extracorporeal blood treatment;
   - calculating, based on the measured initial value of the third parameter (Cd, Na) of the dialysate in the liquid evacuation line (10), an initial value of plasma conductivity ($C_{init}$) and/or an initial value of concentration of at least sodium in plasma ($Na_{init}$);
   - setting the initial value of plasma conductivity ($C_{init}$) as the target plasma conductivity ($C_{target}$) and/or setting the initial value of concentration of at least sodium in plasma ($Na_{init}$) as the target concentration of at least sodium in plasma ($Na_{target}$).

4. The apparatus of claim 3, wherein the procedure for estimating further comprises:

   - measuring at least an initial value of the third parameter (Cd, Na) of the liquid crossing the supply line (9, 11) at the beginning of the extracorporeal blood treatment;
   - calculating the initial value of plasma conductivity ($C_{init}$) and/or the initial value of concentration of at least sodium in plasma ($Na_{init}$) based also on the measured initial value of the third parameter (Cd, Na) of the liquid crossing the supply line (9, 11) or on the initial set point of the third parameter (Cd, Na) of the liquid crossing the supply line (9, 11).

5. The apparatus of claim 3 or 4, wherein the procedure for estimating further comprises:

   - applying a conductivity adjustment factor to the initial value of plasma conductivity ($C_{init}$) to obtain a corrected value of plasma conductivity and/or applying a concentration adjustment factor to the initial value of concentration of at least sodium in plasma ($Na_{init}$) to obtain a corrected value of concentration of at least sodium in plasma; and
   - setting the corrected value of plasma conductivity as the target plasma conductivity ($C_{target}$) and/or setting the corrected value of concentration of at least sodium in plasma as the target concentration of at least sodium in plasma ($Na_{target}$).

6. The apparatus according to claim 3, 4 or 5, wherein the initial set point of the third parameter (Cd, Na) is set by an operator or the control unit (CPU) is configured to calculate the initial set point of the third parameter (Cd, Na) to match a first estimate of the plasma conductivity of the patient (P).

7. The apparatus of claim 6, wherein the control unit (CPU) is configured to calculate the initial set point of the third parameter (Cd, Na) as a function of the type of extracorporeal blood treatment mode.

8. The apparatus of any of claims 3 to 7, wherein the control unit (CPU) is configured to measure a plurality of consecutive initial values of the third parameter (Cd, Na) of the dialysate until said values are stabilized to provide the initial value of the third parameter.

9. The apparatus of any of claims 3 to 8, wherein the procedure for estimating comprises: after calculating the initial value of plasma conductivity ($C_{init}$), the control unit (CPU) is configured to drive a device (30) for regulating a composition of the liquid crossing the supply line to change said composition of the liquid crossing the supply line (9, 11) to reach a liquid conductivity substantially equal to the initial value of plasma conductivity ($C_{init}$).

10. The apparatus of claim 9, wherein the procedure for estimating comprises: after setting the liquid conductivity substantially equal to the initial value of plasma conductivity ($C_{init}$), the control unit (CPU) is configured to execute a second calculating step, based on a second determined initial conductivity of the dialysate and on a second corresponding conductivity of the liquid in the supply line (9, 11), of a second estimate of the initial value of plasma conductivity, said calculating the second estimate being performed maintaining the liquid conductivity substantially constant and substantially equal to the calculated plasma conductivity; wherein, after calculating the second estimate of the initial value of plasma conductivity, the control unit (CPU) is configured to drive the liquid regulating device (30) to change the composition of the liquid and to set the liquid conductivity substantially equal to said second estimate.

11. The apparatus of any of claims 3 to 10, wherein the initial value of concentration of at least sodium in plasma is derived from the initial value of plasma conductivity; wherein the target concentration of sodium in plasma ($Na_{target}$) is set as a concentration of sodium in the liquid crossing the supply line (9, 11) when the liquid conductivity matches the initial value of plasma conductivity.

12. The apparatus according to anyone of the previous claims, wherein the control procedure comprises the following sub-steps:

receiving the target plasma conductivity ($C_{target}$) and/or the target concentration of at least sodium in plasma ($Na_{target}$);
receiving the treatment time (T);
determining, on the basis of the target plasma conductivity ($C_{target}$) and/or on the base of the target concentration of at least sodium in plasma ($Na_{target}$), a third parameter band ($Cd_{band}$, $Na_{band}$) delimited between an upper trajectory and a lower trajectory over the treatment time (T);

wherein the calculated third parameter control values ($Cd_{control(t)}$; $Na_{control(t)}$) are kept within the third parameter band ($Cd_{band}$, $Na_{band}$).

13. The apparatus according to claim 12, wherein the prescription values of the first parameter (BV%) define a first parameter trajectory ($BV\%_{traj(t)}$) and tracking the prescription values of the first parameter (BV%) comprises: keeping or moving the actual values of the first parameter ($BV\%_{meas(t)}$) on said first parameter trajectory ($BV\%_{traj(t)}$) or in a neighborough of said first parameter trajectory ($BV\%_{traj(t)}$); wherein the prescription values of the second parameter (UFR; WLR; WL) define a second parameter band ($UFR_{band}$; $WLR_{band}$; $WL_{band}$) delimited between an upper trajectory and a lower trajectory over the treatment time (T);

wherein calculating the second parameter control value ($UFR_{control(t)}$; $WLR_{control(t)}$, $WL_{control(t)}$) comprises:

determining at least a first error parameter ($ERR\_BV\_UF_{(t)}$) on the basis of:

a difference between an actual value of the first parameter ($BV\%_{meas(t)}$) at the control instant (t) and a corresponding value on the first parameter trajectory ($BV\%_{traj(t)}$); and
a difference between an actual value of the second parameter ($UFR_{meas(t)}$; $WLR_{meas(t)}$; $WL_{meas(t)}$) at the control instant (t) and at least a corresponding value of the second parameter band ($UF1R_{band}$; $WLR_{band}$; $WL_{band}$);

calculating the second parameter control value ($UFR_{control(t)}$; $WLR_{control(t)}$, $WL_{control(t)}$) on the basis of the first error parameter ($ERR\_BV\%\_UF(ty)$) and of the actual value of the second parameter ($UFR_{meas(t-\Delta t)}$; $WLR_{meas(t-\Delta t)}$; $WL_{meas(t-\Delta t)}$)) relating to a preceding control instant;

wherein calculating the third parameter control value ($Cd_{control(t)}$; $Na_{control(t)}$) comprises:

determining at least a second error parameter ($ERR\_BV\_Na(t)$) on the basis of:

a difference between an actual value of the first parameter ($BV\%_{meas(t)}$) at the control instant (t) and a corresponding value on the first parameter trajectory ($BV\%_{traj(t)}$); and
a difference between an actual value of the third parameter ($Cd_{meas(t)}$; $Na_{meas(t)}$) at the control instant (t) and at least a corresponding value of the third parameter band ($Cd_{band}$, $Na_{band}$);

calculating the third parameter control value ($Cd_{control(t)}$; $Na_{control(t)}$) on the basis of the second error parameter ($ERR\_BV\_Na_{(t)}$) and of the actual value of the third parameter ($Cd_{meas(t-\Delta t)}$, $Na_{meas(t-\Delta t)}$) relating

to a preceding control instant.

14. The apparatus according to anyone of the previous claims, wherein, near the end of the extracorporeal blood treatment, the control procedure comprises: driving the third parameter control values ($Cd_{control(t)}$; $Na_{control(t)}$) to converge towards the target plasma conductivity ($C_{target}$) and/or the target concentration of at least sodium in plasma ($Na_{target}$); wherein, at the end of the extracorporeal blood treatment, the control procedure comprises: setting the third parameter control value ($Cd_{control(t)}$; $Na_{control(t)}$) close or substantially equal to the target plasma conductivity ($C_{target}$) and/or the target concentration of at least sodium in plasma ($Na_{target}$).

15. The apparatus according to anyone of the previous claims, wherein the actual values of the third parameter are values of concentration of at least sodium ($Na_{meas(t)}$) in the liquid crossing the supply line (9, 11);

wherein the control procedure uses a mathematical model (M), representing kinetics of the solutes in a distribution volume in the patient, in order to determine equivalent sodium concentration values ($Na_{eq(t)}$), wherein by equivalent sodium concentration at instant t ($Na_{eq(t)}$) is intended the constant sodium concentration in the liquid crossing the supply line (9, 11) which, if it were applied at the start of treatment up to an instant (t), would lead to the same plasma sodium concentration in the patient as is obtained at the same instant (t) with the variation in sodium concentration or conductivity imposed by the control procedure up to time (t);
the control procedure using the equivalent sodium concentration values ($Na_{eq(t)}$) as actual values of the third parameter (Cd, Na) for the determination of the control values.

## Patentansprüche

1. Vorrichtung zur extrakorporalen Blutbehandlung, umfassend:

eine Behandlungseinheit (2) mit einer ersten Kammer (3) und einer zweiten Kammer (4), die durch eine semipermeable Membran (5) voneinander getrennt sind;
eine Blutentfernungsleitung (6), die mit einer Einlassöffnung (3a) der ersten Kammer (3) verbunden und zum Entfernen von Blut von einem Patienten (P) prädisponiert ist,
eine Blutrückführleitung (7), die mit einer Auslassöffnung (3b) der ersten Kammer (3) verbunden und prädisponiert ist, um behandeltes Blut zu dem Patienten (P) zurückzuführen; wobei die Blutentfernungsleitung (6), die Blutrückführleitung (7) und die erste Kammer (3) Teil eines extrakorporalen Blutkreislaufs (8) sind;
wenigstens eine Fluidevakuierungsleitung (10), die mit einer Auslassöffnung (4b) der zweiten Kammer (4) verbunden ist;
eine Zufuhrleitung (9, 11), die mit einer Einlassöffnung (4a) der zweiten Kammer (4) und/oder mit dem extrakorporalen Blutkreislauf (8) verbunden ist;
Sensoreinrichtungen zum Detektieren von Istwerten von Parametern der extrakorporalen Blutbehandlung;
eine Steuereinheit (15), die mit den Sensoreinrichtungen verbunden ist und ausgestaltet ist, um Verordnungswerte der Parameter zu empfangen, die in dem Patienten (P) erreicht werden sollen, oder denen über eine Behandlungszeit (T) gefolgt werden soll, und die Istwerte der Parameter während der extrakorporalen Blutbehandlung durch die Sensoreinrichtungen zu erhalten; wobei die Parameter umfassen:

- einen ersten Parameter (BV%), der sich auf eine Variation des Blutvolumens des Patienten (P) bezieht;
- einen zweiten Parameter (UFR; WLR; WL), der sich auf eine Ultrafiltrationsströmungsrate durch die semipermeable Membran (5) oder auf eine Gewichtsverlustrate des Patienten (P) oder auf einen akkumulierten Gewichtsverlust bezieht;
- einen dritten Parameter (Cd, Na), der aus einer Gruppe ausgewählt wird, die hieraus besteht:

Leitfähigkeit einer die Zufuhrleitung (9, 11) querenden Flüssigkeit, leitfähigkeitsbezogener Parameter der die Zufuhrleitung (9, 11) querenden Flüssigkeit, Konzentration von wenigstens Natrium in der die Zufuhrleitung (9, 11) querenden Flüssigkeit und konzentrationsbezogener Parameter von wenigstens Natrium in der die Zufuhrleitung (9, 11) querenden Flüssigkeit;
wobei die Steuereinheit (15) ausgestaltet ist, um an zeitlich aufeinanderfolgenden Steuerzeitpunkten (t) eine Steuerprozedur durchzuführen, die umfasst:

- Berechnen der folgenden Steuerwerte, die während eines Zeitintervalls ($\Delta t$) nach dem Zeitpunkt (t) festzulegen sind, in dem die Steuerung erfolgt ist, basierend auf den Istwerten und den Verordnungswerten:

eines zweiten Parametersteuerwerts ($UFR_{Steuerung(t)}$; $WLR_{Steuerung(t)}$, $WL_{Steuerung(t)}$) ; und
eines dritten Parametersteuerwerts ($Cd_{Steuerung(t)}$; $Na_{Steuerung(t)}$) ;

- Auferlegen von wenigstens einem von dem zweiten Parametersteuerwert ($UFR_{Steuerung(t)}$; $WLR_{Steuerung(t)}$, $WL_{Steuerung(t)}$) und dem dritten Parametersteuerwert ($Cd_{Steuerung(t)}$; $Na_{Steuerung(t)}$) während des Zeitintervalls ($\Delta t$), das auf den Zeitpunkt (t) folgt, in dem die Steuerung erfolgt ist, sodass die Istwerte des ersten Parameters ($BV\%_{Mess(t)}$) die Verordnungswerte des ersten Parameters ($BV\%_{Traj(t)}$) über die Behandlungszeit (T) verfolgen;

wobei der Verordnungswert des dritten Parameters (Cd, Na) eine Plasma-Zielleitfähigkeit ($C_{Ziel}$) und/oder eine Zielkonzentration von wenigstens Natrium in Plasma ($Na_{ziel}$) umfasst, die in dem Patienten (P) in der Behandlungszeit (T) erreicht werden soll(en); und
**dadurch gekennzeichnet, dass** die Steuereinheit (15) ferner ausgestaltet ist, um wenigstens zu Beginn der extrakorporalen Blutbehandlung eine Prozedur zum Schätzen der Plasma-Zielleitfähigkeit ($C_{Ziel}$) und/oder der Zielkonzentration von wenigstens Natrium in Plasma ($Na_{ziel}$) durchzuführen.

2. Vorrichtung nach Anspruch 1, wobei wenigstens einmal während der extrakorporalen Blutbehandlung die Steuereinheit ausgestaltet ist, um die Plasmaleitfähigkeit und/oder die Konzentration von wenigstens Natrium in Plasma zu prüfen.

3. Vorrichtung nach Anspruch 1 oder 2, umfassend eine Vorbereitungsleitung (18) der die Zufuhrleitung (9, 11) querenden Flüssigkeit, die sich stromaufwärts der Zufuhrleitung (9, 11) befindet und mit dieser verbunden ist; wobei die Prozedur zum Schätzen umfasst:

- Einstellen eines anfänglichen Sollwerts des dritten Parameters der die Zufuhrleitung (9, 11) querenden Flüssigkeit und Vorbereiten einer entsprechenden Flüssigkeit durch die Vorbereitungsleitung (18);
- nach dem Einstellen des anfänglichen Sollwerts, Starten der extrakorporalen Blutbehandlung durch Zirkulieren der Flüssigkeit durch die Zufuhrleitung (9, 11) und durch Zirkulieren von Blut durch den extrakorporalen Blutkreislauf (8);
- Messen eines Anfangswerts des dritten Parameters (Cd, Na) des Dialysats in der Fluidevakuierungsleitung (10) zu Beginn der extrakorporalen Blutbehandlung;
- Berechnen, basierend auf dem gemessenen Anfangswert des dritten Parameters (Cd, Na) des Dialysats in der Fluidevakuierungsleitung (10), eines Anfangswerts von Plasmaleitfähigkeit ($C_{Anfang}$) und/oder eines Anfangswerts einer Konzentration von wenigstens Natrium in Plasma ($Na_{anfang}$) ;
- Einstellen des Anfangswerts der Plasmaleitfähigkeit ($C_{Anfang}$) als Plasma-Zielleitfähigkeit ($C_{Ziel}$) und/oder Einstellen des Anfangswerts einer Konzentration von wenigstens Natrium in Plasma ($Na_{anfang}$) als Zielkonzentration von wenigstens Natrium in Plasma ($Na_{Ziel}$).

4. Vorrichtung nach Anspruch 3, wobei die Prozedur zum Schätzen ferner umfasst:

- Messen wenigstens eines Anfangswerts des dritten Parameters (Cd, Na) der die Zufuhrleitung (9, 11) querenden Flüssigkeit zu Beginn der extrakorporalen Blutbehandlung;
- Berechnen des Anfangswerts von Plasmaleitfähigkeit ($C_{Anfang}$) und/oder des Anfangswerts einer Konzentration von wenigstens Natrium in Plasma ($Na_{anfang}$) außerdem basierend auf dem gemessenen Anfangswert des dritten Parameters (Cd, Na) der die Zufuhrleitung (9, 11) querenden Flüssigkeit oder auf dem anfänglichen Sollwert des dritten Parameters (Cd, Na) der die Zufuhrleitung (9, 11) querenden Flüssigkeit.

5. Vorrichtung nach Anspruch 3 oder 4, wobei die Prozedur zum Schätzen ferner umfasst:

- Anwenden eines Leitfähigkeitsanpassungsfaktors auf den Anfangswert der Plasmaleitfähigkeit ($C_{Anfang}$), um einen korrigierten Wert für die Plasmaleitfähigkeit zu erhalten, und/oder Anwenden eines Konzentrationsanpassungsfaktors auf den Anfangswert der Konzentration von wenigstens Natrium in Plasma ($Na_{Anfang}$), um einen korrigierten Wert der Konzentration von wenigstens Natrium in Plasma zu erhalten; und
- Einstellen des korrigierten Werts von Plasmaleitfähigkeit als Plasma-Zielleitfähigkeit ($C_{Ziel}$) und/oder Einstellen des korrigierten Werts einer Konzentration von wenigstens Natrium in Plasma als Zielkonzentration von wenigstens Natrium in Plasma ($Na_{Ziel}$).

**6.** Vorrichtung nach Anspruch 3, 4 oder 5, wobei der anfängliche Sollwert des dritten Parameters (Cd, Na) durch einen Bediener eingestellt wird oder die Steuereinheit (CPU) ausgestaltet ist, um den anfänglichen Sollwert des dritten Parameters (Cd, Na) zu berechnen, sodass er mit einer ersten Schätzung der Plasmaleitfähigkeit des Patienten (P) übereinstimmt.

**7.** Vorrichtung nach Anspruch 6, wobei die Steuereinheit (CPU) ausgestaltet ist, um den anfänglichen Sollwert des dritten Parameters (Cd, Na) in Abhängigkeit von der Art des extrakorporalen Blutbehandlungsmodus zu berechnen.

**8.** Vorrichtung nach einem der Ansprüche 3 bis 7, wobei die Steuereinheit (CPU) ausgestaltet ist, um mehrere aufeinanderfolgende Anfangswerte des dritten Parameters (Cd, Na) des Dialysats zu messen, bis sich die Werte stabilisiert haben, um den Anfangswert des dritten Parameters bereitzustellen.

**9.** Vorrichtung nach einem der Ansprüche 3 bis 8, wobei die Prozedur zum Schätzen umfasst: nach dem Berechnen des Anfangswerts der Plasmaleitfähigkeit ($C_{Anfang}$) ist die Steuereinheit (CPU) ausgestaltet, um eine Einrichtung (30) zum Regeln einer Zusammensetzung der die Zufuhrleitung querenden Flüssigkeit anzusteuern, um die Zusammensetzung der die Zufuhrleitung (9, 11) querenden Flüssigkeit zu ändern, um eine Flüssigkeitsleitfähigkeit zu erzielen, die im Wesentlichen gleich dem Anfangswert der Plasmaleitfähigkeit ($C_{Anfang}$) ist.

**10.** Vorrichtung nach Anspruch 9, wobei die Prozedur zum Schätzen umfasst: nach dem Einstellen der Flüssigkeitsleitfähigkeit im Wesentlichen gleich dem Anfangswert der Plasmaleitfähigkeit ($C_{Anfang}$) ist die Steuereinheit (CPU) ausgestaltet, um, basierend auf einer zweiten bestimmten anfänglichen Leitfähigkeit des Dialysats und auf einer zweiten entsprechenden Leitfähigkeit der Flüssigkeit in der Zufuhrleitung (9, 11), einen zweiten Berechnungsschritt für eine zweite Schätzung des Anfangswertes der Plasmaleitfähigkeit auszuführen, wobei beim Berechnen der zweiten Schätzung die Flüssigkeitsleitfähigkeit im Wesentlichen konstant und im Wesentlichen gleich der berechneten Plasmaleitfähigkeit gehalten wird; wobei, nach dem Berechnen der zweiten Schätzung des Anfangswerts der Plasmaleitfähigkeit, die Steuereinheit (CPU) ausgestaltet ist, um die Flüssigkeitsregulierungseinrichtung (30) anzusteuern, um die Zusammensetzung der Flüssigkeit zu ändern und die Flüssigkeitsleitfähigkeit im Wesentlichen gleich der zweiten Schätzung einzustellen.

**11.** Vorrichtung nach einem der Ansprüche 3 bis 10, wobei der Anfangswert der Konzentration von wenigstens Natrium in Plasma von dem Anfangswert der Plasmaleitfähigkeit abgeleitet wird; wobei die Zielkonzentration von Natrium in Plasma ($Na_{Ziel}$) als eine Konzentration von Natrium in der die Zufuhrleitung (9, 11) querenden Flüssigkeit eingestellt wird, wenn die Flüssigkeitsleitfähigkeit mit dem Anfangswert der Plasmaleitfähigkeit übereinstimmt.

**12.** Vorrichtung nach einem der vorstehenden Ansprüche, wobei die Steuerprozedur die folgenden Unterschritte umfasst:

Empfangen der Plasma-Zielleitfähigkeit ($C_{Ziel}$) und/oder der Zielkonzentration von wenigstens Natrium in Plasma ($Na_{Ziel}$);
Empfangen der Behandlungszeit (T);
Bestimmen, auf Basis der Plasma-Zielleitfähigkeit ($C_{Ziel}$) und/oder auf Basis der Zielkonzentration von wenigstens Natrium in Plasma ($Na_{Ziel}$), eines dritten Parameterbands ($Cd_{Band}$, $Na_{Band}$), das zwischen einer oberen Trajektorie und einer unteren Trajektorie über die Behandlungszeit (T) begrenzt ist;
wobei die berechneten dritten Parametersteuerwerte ($Cd_{Steuerung(t)}$; $Na_{Steuerung(t)}$) innerhalb des dritten Parameterbands ($Cd_{Band}$, $Na_{Band}$) gehalten werden.

**13.** Vorrichtung nach Anspruch 12, wobei die Verordnungswerte des ersten Parameters (BV%) eine erste Parametertrajektorie ($BV\%_{Traj(t)}$) definieren und das Verfolgen der Verordnungswerte des ersten Parameters (BV%) umfasst: Halten oder Bewegen der Istwerte des ersten Parameters ($BV\%_{Mess(t)}$) auf der ersten Parametertrajektorie ($BV\%_{Traj(t)}$) oder in einer Nachbarschaft der ersten Parametertrajektorie ($BV\%_{Traj(t)}$); wobei die Verordnungswerte des zweiten Parameters (UFR; WLR; WL) ein zweites Parameterband ($UFR_{Band}$; $WLR_{Band}$; $WL_{Band}$) definieren, das zwischen einer oberen Trajektorie und einer unteren Trajektorie über der Behandlungszeit (T) begrenzt ist;
wobei das Berechnen des zweiten Parametersteuerwerts ($UFR_{Steuerung(t)}$; $WLR_{Steuerung(t)}$; $WL_{Steuerung(t)}$) umfasst: Bestimmen wenigstens eines ersten Fehlerparameters ($ERR\_BV\_UF_{(t)}$) basierend auf:

einer Differenz zwischen einem Istwert des ersten Parameters ($BV\%_{Mess(t)}$) zum Steuerzeitpunkt (t) und einem entsprechenden Wert auf der ersten Parametertrajektorie ($BV\%_{Traj(t)}$); und
einer Differenz zwischen einem Istwert des zweiten Parameters ($UFR_{Mess(t)}$ ; $WLR_{Mess(t)}$ ; $WL_{Mess(t)}$) zum Steuerzeitpunkt (t) und wenigstens einem entsprechenden Wert des zweiten Parameterbands ($UFR_{Band}$; $WLR_{Band}$; $WL_{Band}$) ; Berechnen des zweiten Parametersteuerwerts ($UFR_{Steuerung(t)}$; $WLR_{Steuerung(t)}$; $WL_{Steuerung(t)}$) basierend auf dem ersten Fehlerparameter (ERR _BV UF$_{(t)}$) und dem Istwert des zweiten Parameters ($UFR_{Mess(t-\Delta t)}$; $WLR_{Mess(t-\Delta t)}$; $WL_{Mess(t-\Delta t)}$)) in Bezug auf einen vorhergehenden Steuerzeitpunkt.

wobei das Berechnen des dritten Parametersteuerwerts ($Cd_{Steuerung(t)}$; $Na_{Steuerung(t)}$) umfasst:
Bestimmen wenigstens eines zweiten Fehlerparameters (ERR_BV_Na$_{(t)}$) basierend auf:

einer Differenz zwischen einem Istwert des ersten Parameters ($BV\%_{Mess(t)}$) zum Steuerzeitpunkt (t) und einem entsprechenden Wert auf der ersten Parametertrajektorie ($BV\%_{Traj(t)}$) ; und
einer Differenz zwischen einem Istwert des dritten Parameters ($Cd_{Mess(t)}$; $Na_{Mess(t)}$) zum Steuerzeitpunkt (t) und wenigstens einem entsprechenden Wert des dritten Parameterbands ($Cd_{Band}$, $Na_{Band}$) ;
Berechnen des dritten Parametersteuerwerts ($Cd_{Steuerung(t)}$; $Na_{Steuerung(t)}$) basierend auf dem zweiten Fehlerparameter (ERR_BV_Na$_{(t)}$) und dem Istwert des dritten Parameters ($Cd_{Mess(t-\Delta t)}$, $Na_{Mess (t-\Delta t)}$) in Bezug auf einen vorhergehenden Steuerzeitpunkt.

14. Vorrichtung nach einem der vorstehenden Ansprüche, wobei, kurz vor dem Ende der extrakorporalen Blutbehandlung, die Steuerprozedur umfasst: Ansteuern der dritten Parametersteuerwerte ($Cd_{Steuerung(t)}$; $Na_{Steuerung(t)}$) für eine Konvergierung in Richtung der Plasma-Zielleitfähigkeit ($C_{Ziel}$) und/oder der Zielkonzentration von wenigstens Natrium in Plasma ($Na_{ziel}$) ; wobei, am Ende der extrakorporalen Blutbehandlung, die Steuerprozedur umfasst: Einstellen der dritten Parametersteuerwerte ($Cd_{Steuerung(t)}$; $Na_{Steuerung(t)}$) nahe oder im Wesentlichen gleich der Plasma-Zielleitfähigkeit ($C_{Ziel}$) und/oder der Zielkonzentration von wenigstens Natrium in Plasma ($Na_{ziel}$) .

15. Vorrichtung nach einem der vorstehenden Ansprüche, wobei die Istwerte des dritten Parameters Konzentrationswerte von wenigstens Natrium ($Na_{Mess(t)}$) in der die Zufuhrleitung (9, 11) querenden Flüssigkeit sind; wobei die Steuerprozedur ein mathematisches Modell (M) verwendet, das die Kinetik der gelösten Stoffe in einem Distributionsvolumen in dem Patienten darstellt, um äquivalente Natriumkonzentrationswerte ($Na_{Äqu(t)}$) zu bestimmen, wobei durch eine äquivalente Natriumkonzentration zum Zeitpunkt t ($Na_{Äqu(t)}$) die konstante Natriumkonzentration in der die Zufuhrleitung (9, 11) querenden Flüssigkeit beabsichtigt wird, was, falls diese zu Behandlungsbeginn bis zu einem Zeitpunkt (t) angewendet würde, zu derselben Plasma-Natriumkonzentration in dem Patienten führen würde wie sie zu demselben Zeitpunkt (t) mit der Variation der Natriumkonzentration oder Leitfähigkeit erhalten wird, die durch die Steuerprozedur bis zum Zeitpunkt (t) auferlegt wird;
wobei die Steuerprozedur die äquivalenten Natriumkonzentrationswerte ($Na_{Äqu(t)}$) als Istwerte des dritten Parameters (Cd, Na) zur Bestimmung der Steuerwerte verwendet.

**Revendications**

1. Appareil de traitement extracorporel du sang, comprenant :

une unité de traitement (2) comportant une première chambre (3) et une seconde chambre (4) séparées l'une de l'autre par une membrane semi-perméable (5) ;
une ligne de prélèvement de sang (6) reliée à un orifice d'entrée (3a) de la première chambre (3) et prédisposée à prélever le sang d'un patient (P),
une ligne de retour de sang (7) reliée à un orifice de sortie (3b) de la première chambre (3) et prédisposée à renvoyer le sang traité au patient (P) ; la ligne de prélèvement de sang (6), la ligne de retour de sang (7) et la première chambre (3) faisant partie d'un circuit sanguin extracorporel (8) ;
au moins une ligne d'évacuation de liquide (10) reliée à un orifice de sortie (4b) de la seconde chambre (4) ;
une ligne d'alimentation (9, 11) reliée à un orifice d'entrée (4a) de la seconde chambre (4) et/ou reliée au circuit sanguin extracorporel (8) ;
des dispositifs de capteurs pour détecter des valeurs réelles des paramètres du traitement sanguin extracorporel ;
une unité de commande (15) reliée aux dispositifs de capteurs et configurée pour recevoir des valeurs de prescription desdits paramètres à atteindre chez le patient (P) ou à suivre sur un temps de traitement (T) et pour obtenir, par l'intermédiaire des dispositifs de capteurs, les valeurs réelles desdits paramètres au cours du traitement sanguin extracorporel ; lesdits paramètres comprenant :

- un premier paramètre (BV %) relatif à une variation du volume sanguin du patient (P) ;
- un deuxième paramètre (UFR ; WLR ; WL) relatif à un débit d'ultrafiltration à travers la membrane semi-perméable (5) ou à un taux de perte de poids du patient (P) ou à une perte de poids cumulée ;

- un troisième paramètre (Cd, Na) choisi dans un groupe constitué par : la conductivité d'un liquide traversant la ligne d'alimentation (9, 11), un paramètre lié à la conductivité du liquide traversant la ligne d'alimentation (9, 11), la concentration d'au moins le sodium dans le liquide traversant la ligne d'alimentation (9, 11) et un paramètre lié à la concentration d'au moins le sodium dans le liquide traversant la ligne d'alimentation (9, 11) ;

l'unité de commande (15) étant configurée pour réaliser, à des instants de commande temporellement consécutifs (t), une procédure de commande comprenant :

- le calcul, sur la base des valeurs réelles et des valeurs de prescription, des valeurs de commande suivantes à fixer pendant un intervalle de temps ($\Delta t$) après l'instant (t) au cours duquel la commande est effectuée :

    une deuxième valeur de commande de paramètre ($UFR_{control(t)}$; $WLR_{control(t)}$, $WL_{control(t)}$); et
    une troisième valeur de commande de paramètre ($Cd_{control(t)}$ ; $Na_{control(t)}$) ;

- l'imposition d'au moins une de la deuxième valeur de commande de paramètre ($UFR_{control(t)}$; $WLR_{control(t)}$, $WL_{control(t)}$) et de la troisième valeur de commande de paramètre ($Cd_{control(t)}$ ; $Na_{control(t)}$) pendant l'intervalle de temps ($\Delta t$) consécutif à l'instant (t), le contrôle étant effectué de telle sorte que les valeurs réelles du premier paramètre ($BV \%_{meas(t)}$) suivent les valeurs de prescription du premier paramètre ($BV \%_{traj(t)}$) pendant la durée de traitement (T) ;

la valeur de prescription du troisième paramètre (Cd, Na) comprenant une conductivité plasmatique cible ($C_{target}$) et/ou une concentration cible d'au moins le sodium dans le plasma ($Na_{target}$) à atteindre chez le patient (P) pendant le temps de traitement (T) ; et

**caractérisé en ce que** l'unité de commande (15) est en outre configurée pour exécuter, au moins au début du traitement sanguin extracorporel, une procédure d'estimation de ladite conductivité plasmatique cible ($C_{target}$) et/ou de ladite concentration cible d'au moins le sodium dans le plasma ($Na_{target}$).

2. Appareil selon la revendication 1, dans lequel, au moins une fois pendant le traitement extracorporel du sang, l'unité de commande étant configurée pour vérifier la conductivité du plasma et/ou la concentration d'au moins le sodium dans le plasma.

3. Appareil selon la revendication 1 ou 2, comprenant une ligne de préparation (18) du liquide traversant la ligne d'alimentation (9, 11) située en amont de la ligne d'alimentation (9, 11) et connectée à celle-ci ; la procédure d'estimation comprenant :

    - le réglage d'un point de consigne initial du troisième paramètre du liquide traversant la ligne d'alimentation (9, 11) et la préparation d'un liquide correspondant par la ligne de préparation (18) ;
    - après le réglage du point de consigne initial, le démarrage du traitement extracorporel du sang en faisant circuler le liquide dans la ligne d'alimentation (9, 11) et en faisant circuler le sang dans le circuit sanguin extracorporel (8) ;
    - la mesure d'une valeur initiale du troisième paramètre (Cd, Na) du dialysat dans la ligne d'évacuation de liquide (10) au début du traitement extracorporel du sang ;
    - le calcul, sur la base de la valeur initiale mesurée du troisième paramètre (Cd, Na) du dialysat dans la ligne d'évacuation de liquide (10), d'une valeur initiale de conductivité plasmatique ($C_{init}$) et/ou d'une valeur initiale de concentration d'au moins le sodium dans le plasma ($Na_{init}$) ;
    - le réglage de la valeur initiale de la conductivité plasmatique ($C_{init}$) comme conductivité plasmatique cible ($C_{target}$) et/ou le réglage de la valeur initiale de la concentration d'au moins le sodium dans le plasma ($Na_{init}$) comme concentration cible d'au moins le sodium dans le plasma ($Na_{target}$).

4. Appareil selon la revendication 3, la procédure d'estimation comprenant en outre :

    - la mesure d'au moins une valeur initiale du troisième paramètre (Cd, Na) du liquide traversant la ligne d'alimentation (9, 11) au début du traitement extracorporel du sang ;
    - le calcul de la valeur initiale de conductivité plasmatique ($C_{init}$) et/ou de la valeur initiale de concentration d'au moins le sodium dans le plasma ($Na_{init}$) sur la base également de la valeur initiale mesurée du troisième paramètre (Cd, Na) du liquide traversant la ligne d'alimentation (9, 11) ou du point de consigne initial du troisième paramètre (Cd, Na) du liquide traversant la ligne d'alimentation (9, 11).

5. Appareil selon la revendication 3 ou 4, la procédure d'estimation comprenant en outre :

- l'application d'un facteur d'ajustement de conductivité à la valeur initiale de la conductivité plasmatique ($C_{init}$) pour obtenir une valeur corrigée de la conductivité plasmatique et/ou l'application d'un facteur d'ajustement de la concentration à la valeur initiale de la concentration d'au moins le sodium dans le plasma ($Na_{init}$) pour obtenir une valeur corrigée de la concentration d'au moins le sodium dans le plasma ; et
- le réglage de la valeur corrigée de la conductivité plasmatique comme conductivité plasmatique cible ($C_{target}$) et/ou le réglage de la valeur corrigée de la concentration d'au moins le sodium dans le plasma comme concentration cible d'au moins le sodium dans le plasma ($Na_{target}$).

6. Appareil selon la revendication 3, 4 ou 5, le point de consigne initial du troisième paramètre (Cd, Na) étant réglé par un opérateur ou l'unité de commande (CPU) étant configurée pour calculer le point de consigne initial du troisième paramètre (Cd, Na) pour correspondre à une première estimation de la conductivité du plasma du patient (P).

7. Appareil selon la revendication 6, l'unité de commande (CPU) étant configurée pour calculer le point de consigne initial du troisième paramètre (Cd, Na) en fonction du type de mode de traitement extracorporel du sang.

8. Appareil selon l'une quelconque des revendications 3 à 7, l'unité de commande (CPU) étant configurée pour mesurer une pluralité de valeurs initiales consécutives du troisième paramètre (Cd, Na) du dialysat jusqu'à ce que lesdites valeurs soient stabilisées pour fournir la valeur initiale du troisième paramètre.

9. Appareil selon l'une quelconque des revendications 3 à 8, la procédure d'estimation comprenant : après calcul de la valeur initiale de conductivité du plasma ($C_{init}$), l'unité de commande (CPU) étant configurée pour commander un dispositif (30) de régulation d'une composition du liquide traversant la ligne d'alimentation afin de modifier ladite composition du liquide traversant la ligne d'alimentation (9, 11) pour atteindre une conductivité du liquide sensiblement égale à la valeur initiale de conductivité du plasma ($C_{init}$).

10. Appareil selon la revendication 9, la procédure d'estimation comprenant : après avoir établi la conductivité du liquide sensiblement égale à la valeur initiale de conductivité du plasma ($C_{init}$), l'unité de commande (CPU) étant configurée pour exécuter une seconde étape de calcul, sur la base d'une seconde conductivité initiale déterminée du dialysat et d'une seconde conductivité correspondante du liquide dans la ligne d'alimentation (9, 11), d'une seconde estimation de la valeur initiale de conductivité du plasma, ledit calcul de la seconde estimation étant effectué en maintenant la conductivité du liquide sensiblement constante et sensiblement égale à la conductivité du plasma calculée ; après le calcul de la seconde estimation de la valeur initiale de conductivité du plasma, l'unité de commande (CPU) étant configurée pour commander le dispositif de régulation du liquide (30) afin de modifier la composition du liquide et de régler la conductivité du liquide sensiblement égale à ladite seconde estimation.

11. Appareil selon l'une quelconque des revendications 3 à 10, la valeur initiale de concentration d'au moins le sodium dans le plasma étant dérivée de la valeur initiale de conductivité du plasma ; la concentration cible de sodium dans le plasma ($Na_{target}$) étant établie comme une concentration de sodium dans le liquide traversant la ligne d'alimentation (9, 11) lorsque la conductivité du liquide correspond à la valeur initiale de conductivité du plasma.

12. Appareil selon l'une quelconque des revendications précédentes, la procédure de commande comprenant les sous-étapes suivantes :

   la réception de la conductivité plasmatique cible ($C_{target}$) et/ou de la concentration cible d'au moins le sodium dans le plasma ($Na_{target}$) ;
   la réception du temps de traitement (T) ;
   la détermination, sur la base de la conductivité plasmatique cible ($C_{target}$) et/ou sur la base de la concentration plasmatique cible d'au moins le sodium ($Na_{target}$), d'une troisième bande de paramètre ($Cd_{band}$, $Na_{band}$) délimitée entre une trajectoire supérieure et une trajectoire inférieure sur la durée du traitement (T) ;

   les troisièmes valeurs de commande de paramètres calculées ($Cd_{control(t)}$ ; $Na_{control(t)}$) étant maintenues dans la troisième bande de paramètre ($Cd_{band}$, $Na_{band}$).

13. Appareil selon la revendication 12, les valeurs de prescription du premier paramètre (BV %) définissent une première trajectoire de paramètre ($BV\%_{traj}(t)$) et le suivi des valeurs de prescription du premier paramètre (BV %) comprenant : le maintien ou le déplacement des valeurs réelles du premier paramètre ($BV\%_{meas(t)}$) sur ladite première trajectoire de paramètre ($BV\%_{traj(t)}$) ou dans un voisinage de ladite première trajectoire de paramètre ($BV\%_{traj(t)}$) ; les valeurs de prescription du deuxième paramètre (UFR ; WLR ; WL) définissant une deuxième bande de paramètre ($UFR_{band}$ ;

$WLR_{band}$; $WL_{band}$) délimitée entre une trajectoire supérieure et une trajectoire inférieure sur la durée de traitement (T) ; le calcul de la deuxième valeur de commande de paramètre ($UFR_{control(t)}$; $WLR_{control(t)}$, $WL_{control(t)}$) comprenant :

la détermination d'au moins un premier paramètre d'erreur ($ERR\_BV\_UF_{(t)}$) sur la base de :

une différence entre une valeur réelle du premier paramètre ($BV\%_{meas(t)}$) à l'instant de commande (t) et une valeur correspondante sur la première trajectoire de paramètre ($BV\%_{traj(t)}$) ; et
une différence entre une valeur réelle du deuxième paramètre ($UFR_{meas(t)}$ ; $WLR_{meas(t)}$ ; $WL_{meas(t)}$) à l'instant de commande (t) et au moins une valeur correspondante de la deuxième bande de paramètre ($UFR_{band}$; $WLR_{band}$; $WL_{band}$) ;

le calcul de la deuxième valeur de commande de paramètre ($UFR_{control(t)}$; $WLR_{control(t)}$, $WL_{control(t)}$) sur la base du premier paramètre d'erreur ($ERR\_BV\%\_UF_{(t)}$), et de la valeur réelle du deuxième paramètre ($UFR_{meas(t-\Delta t)}$; $WLR_{meas(t-\Delta t)}$; $WL_{meas(t-\Delta t)}$) relative à un instant de commande précédent ;

le calcul de la troisième valeur de commande de paramètre ($Cd_{control(t)}$ ; $Na_{control(t)}$) comprenant :

la détermination d'au moins un deuxième paramètre d'erreur ($ERR\_BV\_Na_{(t)}$) sur la base de :

une différence entre une valeur réelle du premier paramètre ($BV\%_{meas(t)}$) à l'instant de commande (t) et une valeur correspondante sur la première trajectoire de paramètre ($BV\%_{traj(t)}$); et
une différence entre une valeur réelle du troisième paramètre ($Cd_{meas(t)}$; $Na_{meas(t)}$) à l'instant de commande (t) et au moins une valeur correspondante de la troisième bande de paramètre ($Cd_{band}$, $Na_{band}$) ;

le calcul de la troisième valeur de commande de paramètre ($Cd_{control(t)}$ ; $Na_{control(t)}$) sur la base du deuxième paramètre d'erreur ($ERR\_BV\_Na_{(t)}$), et de la valeur réelle du troisième paramètre ($Cd_{meas(t-\Delta t)}$, $Na_{meas(t-\Delta t)}$) relative à un instant de commande précédent.

14. Appareil selon l'une quelconque des revendications précédentes, près de la fin du traitement sanguin extracorporel, la procédure de commande comprenant : la commande des troisièmes valeurs de commande de paramètres ($Cd_{control(t)}$ ; $Na_{control(t)}$) pour converger vers la conductivité plasmatique cible ($C_{target}$) et/ou la concentration cible d'au moins le sodium dans le plasma ($Na_{target}$) ; à la fin du traitement sanguin extracorporel, la procédure de commande comprenant : le réglage de la troisième valeur de commande de paramètre ($Cd_{control(t)}$ ; $Na_{control(t)}$) proche ou sensiblement égale à la conductivité plasmatique cible ($C_{target}$) et/ou à la concentration cible d'au moins le sodium dans le plasma ($Na_{target}$) .

15. Appareil selon l'une quelconque des revendications précédentes, les valeurs réelles du troisième paramètre étant des valeurs de concentration d'au moins le sodium ($Na_{meas(t)}$) dans le liquide traversant la ligne d'alimentation (9, 11) ;

la procédure de commande utilisant un modèle mathématique (M) représentant la cinétique des solutés dans un volume de distribution chez le patient, pour déterminer des valeurs de concentration en sodium équivalentes ($Na_{eq(t)}$), la concentration en sodium équivalente à l'instant t ($Na_{eq(t)}$) étant la concentration constante en sodium dans le liquide traversant la ligne d'alimentation (9, 11) qui, si elle était appliquée au début du traitement jusqu'à un instant (t), conduirait à la même concentration plasmatique de sodium chez le patient que celle obtenue au même instant (t) avec la variation de la concentration ou de la conductivité de sodium imposée par la procédure de commande jusqu'au temps (t) ;
la procédure de commande utilisant les valeurs de concentration équivalente en sodium ($Na_{eq(t)}$) comme valeurs réelles du troisième paramètre (Cd, Na) pour la détermination des valeurs de commande.

FIG.1

FIG.2

FIG.3A

FIG.3B

EP 4 098 291 B1

FIG.3C

EP 4 098 291 B1

## FIG.4

PROCEDURE FOR ESTIMATING TARGETS

- SETTING AN INITIAL SET POINT OF LIQUID CONDUCTIVITY OR SODIUM CONCENTRATION

- PREPARING A CORRESPONDING LIQUID

- STARTING THE EXTRACORPOREAL BLOOD TREATMENT

- MEASURING INITIAL CONDUCTIVITY OF THE LIQUID CROSSING THE SUPPLY LINE

- MEASURING INITIAL CONDUCTIVITY OF THE DIALYSATE DOWNSTREAM OF THE SECOND CHAMBER

- CALCULATING INITIAL VALUE OF PLASMA CONDUCTIVITY OR INITIAL VALUE OF CONCENTRATION OF SODIUM IN PLASMA

- SETTING THE INITIAL VALUE OF PLASMA CONDUCTIVITY AS THE TARGET PLASMA CONDUCTIVITY $(Cd_{target})$ OR SETTING THE INITIAL VALUE OF CONCENTRATION OF SODIUM IN PLASMA AS THE TARGET CONCENTRATION OF SODIUM IN PLASMA $(Na_{target})$

- STARTING CONTROL PROCEDURE

## FIG.5

CONTROL PROCEDURE

- RECEIVING TARGET PLASMA CONDUCTIVITY $(Cd_{target})$ OR TARGET CONCENTRATION OF SODIUM IN PLASMA $(Na_{target})$ AND OTHER PRESCRIPTION VALUES

- MEASURING ACTUAL VALUES

- CALCULATING CONTROL VALUES, ON THE BASIS OF THE ACTUAL VALUES AND OF THE PRESCRIPTION VALUES

- IMPOSING THE CONTROL VALUES SUCH THAT THE ACTUAL VALUES TRACK THE PRESCRIPTION VALUES OVER TREATMENT TIME

- DRIVING THE CONTROL VALUES TO CONVERGE TOWARDS THE TARGET PLASMA CONDUCTIVITY $(C_{target})$ AND/OR THE TARGET CONCENTRATION OF SODIUM IN PLASMA $(Na_{target})$ AT THE END OF THE EXTRACORPOREAL BLOOD TREATMENT

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012172398 A1
- WO 2012172398 A

- WO 2018095694 A1